Europäisches Patentamt

European Patent Office

Office européen des brevets

(11) Publication number: **0 222 509**
**A2**

(12) # EUROPEAN PATENT APPLICATION

(21) Application number: 86307863.0

(51) Int. Cl.⁴: **A61F 2/20**

(22) Date of filing: 10.10.86

(30) Priority: 11.10.85 US 786508
07.08.86 US 894274

(43) Date of publication of application:
20.05.87 Bulletin 87/21

(84) Designated Contracting States:
DE FR GB IT

(71) Applicant: HANSA MEDICAL PRODUCTS INC.
1633 North Capitol Avenue
Indianapolis Indiana 46202(US)

(72) Inventor: Blom, Eric D.
8939 Shagbark Road
Indianapolis Indiana 46260(US)
Inventor: Singer, Mark I.
1158 W 106th Street
Carmel Indiana 46032(US)

(74) Representative: Allen, William Guy Fairfax et
al
J.A. KEMP & CO. 14 South Square Gray's Inn
London WC1R 5EU(GB)

(54) **Prosthetic device for use in voice restoration.**

(57) A prosthetic device for use in voice restoration in which a cylindrical body (32) of relatively rigid material is construction of a size to be positioned substantially permanently in a fistula between a patient's trachea and esophagus, means (52,54) being provided to retain the cylindrical body in the fistula. A voice prosthesis (26) having a passageway (24) at one end and a valve adjacent the other end (28) can be inserted and removed from the cylindrical body - (32) without the need to remove the cylindrical body, thereby facilitating cleaning or replacement.

FIG. 2

EP 0 222 509 A2

## PROSTHETIC DEVICE FOR USE IN VOICE RESTORATION

The present invention relates to a prosthetic device for use in voice restoration.

A known form of device is disclosed in US-A-4435853. Such a device comprises a voice prosthesis including a cylinder, means for providing a passageway for the entry of air at one end of the cylinder, a one way valve adjacent to the other end of the cylinder and means for retaining the cylinder in a fistula formed between the patient's trachea and esophagus.

Voice prosthesis devices of this type are generally constructed from relatively soft, pliable, biocompatible materials. These devices become contaminated with food particles, mucus, saliva and certain species of Candida, flora that occasionally infect mucus membranes of the throat and trachea, and it is difficult to clean or replace these devices.

It is now proposed, according to one aspect of the present invention, to provide a prosthetic device for use in voice restoration, said device comprising a cylindrical body of relatively rigid material of a size to be positioned substantially permanently in a fistula, between a patient's trachae and esophagus, and means for retaining the cylindrical body in the fistula.

The cylinder of a voice prosthesis can be slidably received in the cylindrical body, so that the body can remain in the fistula and the voice prosthesis can be readily removed for cleaning or replacement.

In order to clean the voice prosthesis, it is not uncommon for the prosthesis to be out of the user's fistula for a period of up to five to ten minutes, during which time the cylindrical body can remain in place in order to maintain the fistula. If one fails to maintain the fistula at all times, it will close rapidly, even in as short a time as five to ten minutes, to the point that the patient would not be able to get the device back into the fistula. This would require that another surgical operation be performed to reopen the fistula so that the cleaned voice prosthesis or a new voice prosthesis can be inserted. Some surgeons have expressed a reluctance to use voice prosthesis devices of these general types, because of this propensity of the fistula to reclose when the voice prosthesis device is removed for regular hygiene, repair or replacement.

Furthermore, hitherto many patients Have found that it is sometimes difficult to replace the voice prosthesis device in the fistula even when the voice prosthesis device is only out for a relatively shorter period of time than that necessary for the fistula to close completely. The fistula prolapses essentially immediately when the voice prosthesis device is removed, and so is somewhat smaller even when the device has not been out of the fistula for too long a period of time. The construction of the present invention overcomes these problems.

As used herein, the terms "cylinder" and "cylindrical body" have their conventional mathematical definition, that is, a body bounded by two parallel planes (the ends of the cylinder) and a surface whose directrix (its projection on either of the two parallel planes) is closed.

Preferably the cylindrical body is constructed of a biocompatible material such as stainless steel or silicone. The means for retaining the cylinder in the fistula may comprise at least one radial projection, for example spaced first and second projections, such as flanges, extending radially outwardly from the exterior wall of the cylinder. When the cylindrical body is positioned in the fistula, the fist projection lies adjacent the anterior wall of the esophagus and the second projection lies adjacent to the posterior wall of the trachea.

In one construction, the cylindrical body comprises an outer cylindrical wall for contacting the tissue of the fistula and an inner cylindrical wall having a first portion of a relatively large size for positioning towards the esophageal end of the fistula and a second portion of a relatively smaller size for positioning towards the tracheal end of the fistula. A transition portion of the inner cylindrical wall lies between the first and second portions and may comprise a tapered or a curved region between the first, larger sized portion and the second, smaller sized portion.

According to another aspect of the invention, there is provided a prosthetic device for use in voice restoration, said device comprising a voice prosthesis including a cylinder, means for providing a passageway for the entry of air into the cylinder adjacent one end thereof, a one way valve adjacent the other end thereof, and means for retaining the cylinder in the fistula between a patient's trachea and esophagus, characterised in that the retaining means comprises a cylindrical body of relatively rigid material of a size to be positioned substantially permanently in said fistula, means for retaining the implant in the fistula and in that the cylinder is slidably receivably in said cylindrical body.

If the cylindrical body used in the present invention is formed of silicone, the cylinder of the voice prosthesis is preferably formed of a relatively softer, more pliable and resilient silicone and the cylindrical body portion comprises relatively harder, less pliable and more rigid silicone.

The cylindrical body may comprise a first cylindrical body portion having a radially outwardly extending projection at one end, a second cylindrical body portion having a radially outwardly extending projection at its end and co-operating threads on said cylindrical body portions allowing said body portions to be threadably engaged with one another. This threaded engagement can be carried out during the fitting of the cylindrical body to the fistula.

The invention may best be understood by referring to the following description and accompanying drawings which illustrate the invention. In the drawings:

Figure 1 is a partly cut away side elevational view of a portion of a human head and neck showing the location of the device of the present invention;

Figure 2 is a greatly enlarged sectional side elevational view of a detail of Figure 1;

Figure 3 is an enlarged sectional side elevational view of an alternative construction of the device of Figure 2;

Figure 4 is an enlarged sectional side elevational view of another construction of the device of Figure 2, showing a tool and spacer for use with the devices of Figures 1-4; and

Figure 5 illustrates an enlarged sectional side elevational view of another construction of the device of Figures 2-4.

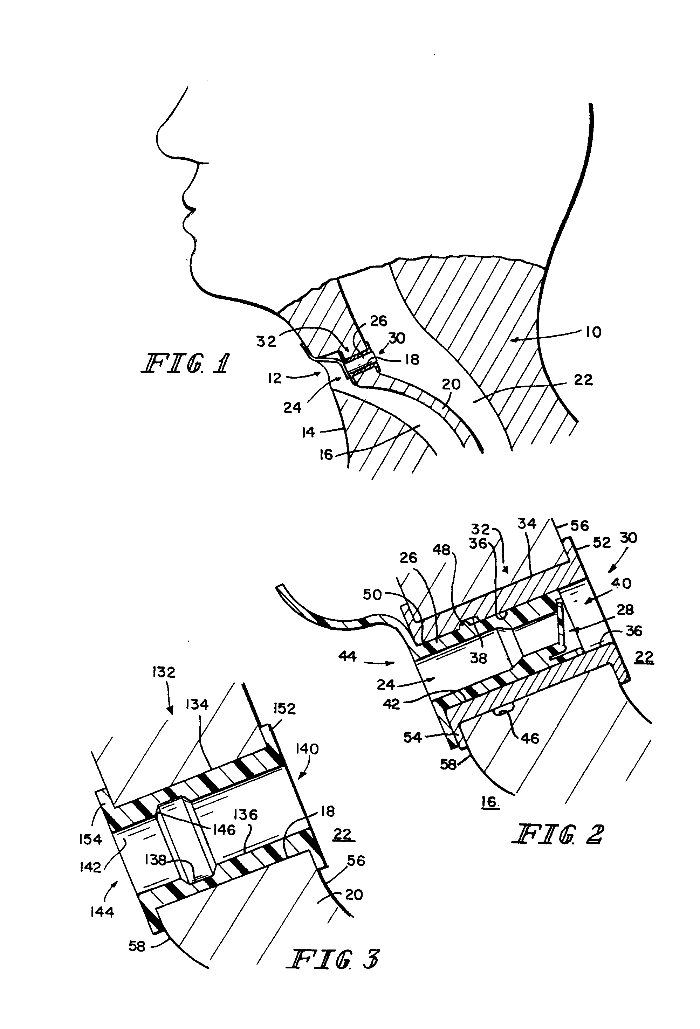

Turning now to Figure 1, a laryngectomee 10 is provided with a tracheostoma 12 opening from the outside of his neck 14 to his trachea 16. In an operation to restore his voice, a puncture establishes a fistula 18 through the wall 20 of tissue that separates his trachea 16 from his esophagus 22. In the prior art, a voice prosthesis device of the general type of those noted above was inserted into the fistula 18. When the laryngectomee 10 desired to speak, he had only to occlude his tracheostoma 12 with his finger or through the action of a device of the type described in US-A-4435853. This permitted air from the trachea 16 to be channeled into one end 24 of the voice prosthesis device 26, through the one-way valve 28 adjacent the other end 30 of device 26 and into the esophagus 22, permitting esophageal speech.

According to the invention, to facilitate hygiene and replacement of the device 26, a surgical implant 32 in the form of a cylindrical body is positioned in the fistula 18 when the fistula 18 is originally formed or punctured through the wall 20. The cylindrical body 32 comprises an outer cylindrical wall 34 and an inner cylindrical wall 36. The inner cylindrical wall 26 is divided into a first, larger diameter portion 38 positioned toward the esophageal end 40 and a second, smaller diameter portion 42 positioned toward the trachael end 44 of the implant 32. Larger diameter portion 38 illustratively is in the form of an annular groove which extends around wall 36. An intermediate portion 46 between portions 38 and 42 is formed as a curved region 46. This intermediate portion co-operates with a radially outwardly extending projection 48 formed on the outer cylindrical side wall 50 of voice prosthesis device 26 to secure device 26 within implant 32. Because device 26 is typically constructed from a relatively soft, pliable and resilient silicone material, it is relatively simple to snap device 26 into and out of engagement in implant 32 for hygiene and replacement purposes.

A radially outwardly extending flange 52 is provided at the esophageal end 40 of surgical implant 32, and a radially outwardly extending flange 54 is provided at the tracheal end 44 of implant 32. When implant 32 is positioned in the fistula, flange 52 lies adjacent the anterior wall 56 of the esophagus 22 and flange 54 lies adjacent the posterior wall 58 of the trachea 16.

In the embodiment of Figure 3, a surgical implant in the form of a cylindrical body 132 is positioned in the fistula 18 when the fistula 18 is originally formed or punctured through the wall 20. The cylindrical body 132 comprises an outer cylindrical wall 134 and an inner cylindrical wall 136. The inner cylindrical wall 136 is divided into a first, larger diameter portion 138 positioned toward the esophageal end 140 and a second, smaller diameter portion 142 positioned toward the tracheal end 144 of the implant 132. Larger diameter portion 138 illustratively is in the form of an annular groove which extends around wall 136. An intermediate portion 146 between portions 138 and 142 is formed as a tapered region 146. This intermediate portion co-operates with the radially outwardly extending projection 48 formed on the outer cylindrical side wall 50 of voice prosthesis device 26 to secure device 26 within implant 132. Because device 26 is typically constructed from a relatively soft, pliable and resilient silicone material, it is relatively simple to snap device 26 into and out of engagement in implant 132 for hygiene and replacement purposes.

A radially outwardly extending flange 152 is provided at the esophageal end 140 of cylindrical body 132, and a radially outwardly extending flange 154 is provided at the tracheal end 144 of implant 132. When cylindrical bdoy 132 is positioned in the fistula, flange 152 lies adjacent the anterior wall 56 of the esophagus 22 and flange 154 lies adjacent the posterior wall 58 of the trachea 16.

There may be some edema of the wall 20 as a result of placement of the cylindrical body 32 or 132. Referring to Figure 4, to compensate for this edema, a tool 160 and resilient rings 162 of a biocompatible material such as a soft silicone are provided. The rings 162 are expanded and slipped over the enlarged portion 164 of the tool adjacent its end 166 remote from its handle (not shown). The remote end 166 is slipped into the cylindrical body 32, 132 and a ring 162 is worked off the enlarged portion 164, which has the same size as the flange 54 or 154, over the flange 54, 154 and onto the exterior wall 34, 134 of the cylindrical body 32, 132. Ring 162 acts as a spacer between the flange 54, 154 and a posterior wall 58 of the trachea to prevent cylindrical body. 32, 132 from sliding back and forth in fistula 18 after the edema has subsided. Because there may be varying degrees of edema, different thickness rings 162 can be provided.

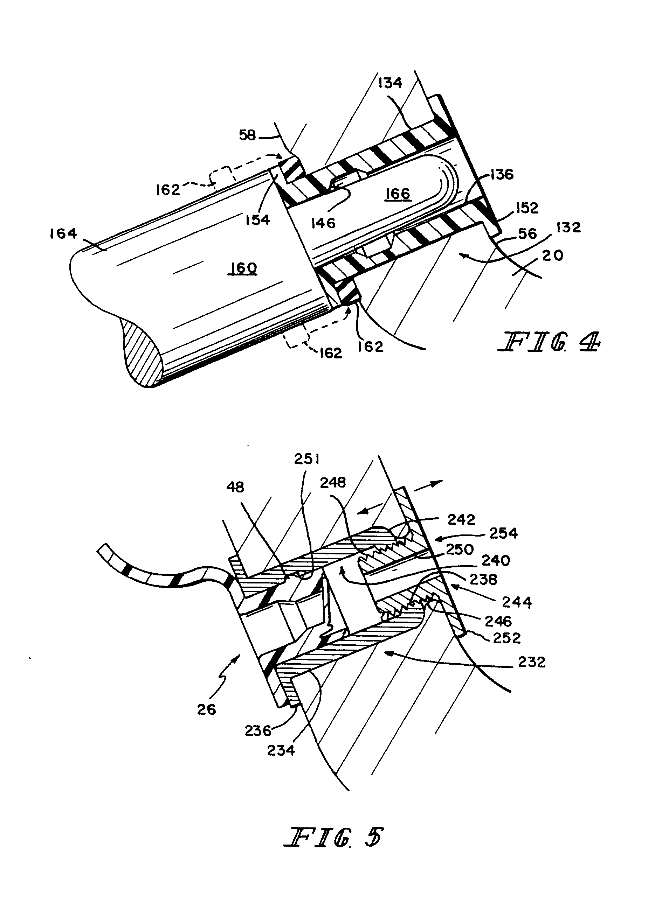

In the embodiment of the invention illustrated in Figure 5, an implant 232 comprises a fist cylindrical female member 234 having a radially outwardly extending flange 236 at one end and an interiorly threaded 238 right circular cylingrical passageway 240 extending through it from its flange 236 end to its other end 242. Although passageway 240 extends completely through to the flanged 236 end, the threads 238 stop a short distance from end 242. Implant 232 also includes a male member 244 having a right circular cylindrical outer sidewall 246 which has threads 248 complementary to threads 238. Male member 244 includes a central passageway 250 and a radially outwardly extending flange 252 at its end 254. Passageway 250 is sized to receive a voice prosthesis device such as device 26 of Figures 1 and 2. The voice prosthesis device can be designed with a radial projection such as projection 48 of Figures 1 and 2, and a radial indentation 251 is provided in the passageway 250.

The embodiment of Figure 5 is intended for use in those situations in which the thickness of the tracheoesophageal wall changes while the implant is in the tracheoesophageal puncture. If that happens, the male member 244 can be screwed into or out of the female member 234, depending upon whether the thickness of the tracheoesophageal wall around the puncture is decreasing or increasing. In this orientation, the distance between flanges 236, 252 equals the tracheoesophageal wall thickness.

**Claims**

1. A prosthetic device for use in voice restoration, said device comprising a cylindrical body (32, 132,232) of relatively rigid material of a size to be positioned substantially permanently in a fistula - (18), between a patient's trachea (16) and esophagus (22), and means (52,54,152,154,236,252) for retaining the cylindrical body in the fistula.

2. A prosthetic device for use in voice restoration, said device comprising a voice prosthesis including a cylinder (26), means for providing a passageway for the entry of air into the cylinder - (26) adjacent one end (24) thereof, a one way valve (28) adjacent the other end (30) thereof, and means for retaining the cylinder in a fistula (18) between a patient's trachae (16) and esophagus (22), characterised in that said retaining means comprises a cylindrical body (32,132,232) of relatively rigid material of a size to be positioned substantially permanently in said fistula (18), means (52,54,152,154,236, 252) for retaining the implant in the fistula and in that the cylinder (26) is slidably receivable in said cylindrical body.

3. A device according to claim 2, characterised in that said cylinder (26) includes a projection (48) between its ends (24,30) in that the cylindrical body includes an outer surface (34,44,234) for contacting the tissue of the fistula (18) and an inner cylindrical wall (36,136,240) including means - (38,238,251) for engaging the projection on the cylinder (26) to retain the cylinder (26) in the cylindrical body (32,132,232).

4. A device according to claim 3, characterised in that said means for engaging the radial projection comprise a first portion (38,138,251) of relatively large size, a second portion (36,136,240) of relatively smaller size, and a transition connection - (46,146) between the first and second portions, the first portion lying towards the esophageal end of the cylindrical body and the second portion lying towards the tracheal end of the cylindrical body, the projection on the cylinder lying in the first portion when, the voice prosthesis is slidably received in said cylindrical body to retain it in position therein.

5. A device according to claim 4, characterised in that the first portion (38,138,251) is in the form of an angular groove on the inner surface of the cylindrical body (32,132,232).

6. A device according to claim 4 or 5, characterised in that the transition connection (46,146) is tapered or curved.

7. A device according to any preceding claim, characterised in that the means for retaining the cylindrical body in the fistula comprise at least one radial projection (52,54,152,154,236,254) projecting outwardly from said cylindrical body.

8. A device according to claim 7, characterised in that said at least one radial projection comprises a radial flange at each end of the cylindrical body.

9. A device according to any preceding claim, wherein the cylindrical body is constructed of stainless steel.

10. A device according to any preceding claim, wherein the cylindrical body is constructed of silicon.

11. A device according to claim 10 when appendant to claim 2, characterised in that the cylinder is formed of a relatively softer, more pliable and resilient silicone and the cylindrical body comprises relatively harder, less pliable and more rigid silicone.

12. A device according to any preceding claim, characterised in that the cylindrical body (232) comprises a first cylindrical body portion (234) having a radially outwardly extending projection - (236) at one end, a second cylindrical body portion (244) having a radially outwardly extending projection (252) at its end, and co-operating threads - (238,248) on said cylindrical body portions allowing said portions to be threadably engaged with one another.

FIG. 1

FIG. 2

FIG. 3

FIG. 4

FIG. 5